# EUROPEAN PATENT SPECIFICATION

(11) **EP 4 489 729 B1**
(45) Date of publication and mention of the grant of the patent: **09.09.2026**
(21) Application number: 23710339.5
(22) Date of filing: 09.03.2023
(51) Int. Cl.: A61K 9/107, A61K 9/19

(54) **EMULSION FOR NON-INVASIVE BLOOD-BRAIN BARRIER TRANSIENT OPENING AND CONTROLLED DRUG RELEASE INTO THE BRAIN**
EMULSION ZUR NICHT-INVASIVEN VORÜBERGEHENDEN ÖFFNUNG DER BLUT-HIRN-SCHRANKE UND ZUR KONTROLLIERTEN ARZNEIMITTELABGABE IN DAS GEHIRN
ÉMULSION POUR L'OUVERTURE TRANSITOIRE NON INVASIVE DE LA BARRIÈRE HÉMATO-ENCÉPHALIQUE ET LA LIBÉRATION CONTRÔLÉE DE MÉDICAMENTS DANS LE CERVEAU

(30) Priority: 11.03.2022 EP 22305284
(43) Date of publication of application: 15.01.2025
(73) Proprietor: Avignon Université, 84029 Avignon (FR); Centre National de la Recherche Scientifique, 75016 Paris (FR); INSTITUT NATIONAL DE LA SANTE ET DE LA RECHERCHE MEDICALE - INSERM, 75013 Paris (FR); Sorbonne Université, 75006 Paris (FR); Université de Tours, 37000 Tours (FR); Université de Paris Cité, 75006 Paris (FR)
(72) Inventor: URBACH, Wlodzimierz, 75020 PARIS (FR); DESGRANGES, Stéphane, 84000 AVIGNON (FR); RAMESH, Rashmi, 93110 ROSNY SOUS BOIS (FR); TAULIER, Nicolas, 75014 PARIS (FR); ESCOFFRE, Jean-Michel, 37200 TOURS (FR); CONTINO-PEPIN, Christiane, 84210 Althen-des-Paluds (FR)
(74) Representative: Germain Maureau
(86) International application number: PCT/EP2023/056066
(87) International publication number: WO 2023/170231

(56) References cited:
- EP-A1- 3 095 806
- US-A1- 2021 252 172
- CALDERÓ GABRIELA ET AL: "Ultrasound/radiation-responsive emulsions", CURRENT OPINION IN COLLOID & INTERFACE SCIENCE, LONDON, GB, vol. 49, 20 August 2020 (2020-08-20), pages 118 - 132, XP086322967, ISSN: 1359-0294, [retrieved on 20200820], DOI: 10.1016/J.COCIS.2020.08.002
- K. ASTAFYEVA ET AL: "Perfluorocarbon nanodroplets stabilized by fluorinated surfactants: characterization and potentiality as theranostic agents", JOURNAL OF MATERIALS CHEMISTRY. B, vol. 3, no. 14, 1 January 2015 (2015-01-01), GB, pages 2892 - 2907, XP055284741, ISSN: 2050-750X, DOI: 10.1039/C4TB01578A
- BOWEN P: "Particle Size Distribution Measurement from Millimeters to Nanometers and from Rods to Platelets", JOURNAL OF DISPERSION SCIENCE AND TECHNOLOGY, TAYLOR AND FRANCIS GROUP, NEW YORK, NY, US, vol. 23, no. 5, 1 January 2002 (2002-01-01), pages 631 - 662, XP009102859, ISSN: 0193-2691, DOI: 10.1081/DIS-120015368

## Description

### TECHNICAL FIELD

The present disclosure concerns, but is not limited to, an emulsion, such as, for example, an injectable emulsion, and an emulsion for use, *e.g.,* in brain therapy. The present disclosure also concerns a dry formulation obtained from the emulsion, a dry formulation for use in brain therapy, as well as a method for delivering a drug into a brain tissue.

### TECHNICAL BACKGROUND

Brain disorders such as those caused by tumors, neurodegenerative and neuropsychiatric diseases, trauma, vascular injury or epilepsy, affect today one out of three people worldwide, thus representing a serious public health problem. Crossing the Blood-Brain Barrier (BBB) remains a challenge for a successful treatment of diseases affecting the Central Nervous System (CNS), for example when the therapy thereof involves hydrophilic molecules of molecular weight greater than 400 Da. In the absence of specific mechanisms such as membrane transporters or endocytosis, these molecules are unable to cross the BBB. In the attempt of enhancing the brain uptake of these molecules by passive diffusion across the BBB, several drugs have been chemically modified by reducing hydrogen bonding, thereby increasing their lipophilicity. However, a large number of lipophilic compounds are rapidly pumped back into bloodstream by BBB-related drug efflux transporters, which reduces the efficiency of lipophilic drugs. Therefore, most potentially promising drugs under development may never reach the clinics.

Focused Ultrasound (FUS) using the interaction between the pressure field and microbubbles (MBs) to cause the transient opening of the BBB has emerged as a promising approach [Hynynen et al, Acta Neurochir. Suppl. (Wien) 86, 555-558 (2003)]. MBs are perfluorocarbon gas-filled bubbles, which may for example be coated by polymeric, protein or lipid shells. MBs are injected intravenously and circulate in the vascular system just before exposure to US, or "insonation". It is known that the stable oscillation of MBs stimulated by ultrasonic energy induces mechanical stress and transient down-expression of proteins which maintain the tight junction between endothelial cells ensuring the permeability of BBB. When associated with magnetic resonance guidance, FUS appears as a new non-invasive surgical modality allowing a safe and transient BBB opening with a high degree of spatial and temporal specificity *(i.e.,* permitting sub-millimetric precision and restoring the normal barrier function after a few hours, respectively). The safety and reversibility of US-induced BBB opening have been proven using a transcranial device in patients with malignant brain tumors or suffering from neurodegenerative disorders such as Alzheimer's disease or amyotrophic lateral sclerosis. Currently, eight ongoing clinical trials are running worldwide. These trials are focused on determining the safety of treatments using FUS in combination with MBs in a variety of pathological contexts, including glioblastoma, Alzheimer's disease, Parkinson's disease, and amyotrophic lateral sclerosis.

All these clinical trials are performed under FUS combined with intravenously injected MB contrast agents circulating in the vasculature just before US exposure.

Efficient and safe FUS-mediated BBB opening depends on a number of parameters including MB size and dose, volume and type of targeted tissue, MB handling and coordination between insonation and MB injection. Although longer insonation times seem to increase the magnitude of BBB disruption, insonation time longer than the lifetime of MBs in the blood is useless and can lead to brain damages.

Over the last decade, many studies about the US-induced BBB opening and drug delivery to the brain parenchyma were reported in the literature. Examples of this combination of US-induced BBB opening and drug delivery are disclosed in [Chen, H. et al. J. Cereb. Blood Flow Metab. (2014)], which is directed to the delivery of low molecular weight therapeutic agents or fluorescently labeled dextrans of variable hydrodynamic diameters (from 2.3 to 54.4 nm) using commercially available MBs *(e.g.,* Definity^{®}, SonoVue^{®}). Although efficient BBB-disruption was achieved, MBs are constrained within the intravascular space due to their micron-sized diameter, thus causing, as a drawback, an action limited to regions at or near the vessels. An additional drawback of commercial MBs is their fast disappearance (within 15 minutes), as the bubble gas encapsulated in their core is rapidly dissolved into the plasma.

For these reasons, a growing interest is given to Acoustic Droplet Vaporization (ADV) of perfluorocarbon "droplets" (PFCD) e.g. micron or nano-sized, which remain in the vasculature for more than 2 hours after intravenous injection. Liquid perfluorocarbons in the form of droplets are more stable than their resulting bubbles over time while still offering the possibility to disrupt the BBB after vaporization and bubble oscillation for drug delivery purposes [Zhou, Y. J. Ther. Ultrasound 3, 20 (2015); Fabiilli, M. L. et al. IEEE Trans. Ultrason. Ferroelectr. Freq. Control 56, 1006-1017 (2009)]. However, ADV is far from being completely understood and the exact conditions leading to droplet vaporization cannot be easily predicted. Moreover, PFCD disclosed in the literature are poorly characterized and their size is largely polydisperse, which is not adequate for precise ADV. Even if the above approaches can permeabilize the BBB and improve the passage of drugs across the brain endothelium, once the drugs reach the cerebrospinal fluids, some drugs with intracellular site of action are still confronted with the membrane of CNS cells, which drastically diminishes their efficiency. In addition, since only a small amount of the administered free drug effectively crosses the opened BBB, many CNS drugs may induce severe side effects.

Hence, in most reported studies, the drug or model drug is co-injected in free form with the MB or nanodroplet intended to open the BBB with no assurance, however, of sufficient drug accumulation in the brain tissue [Bensimon, G. et al. N. Engl. J. Med. 330, 585-591 (1994); Wu, S.Y. et al. Phys Med Biol. 63 (3), 035002 (2018)].

Despite the efforts made by the scientific community to develop nanocarriers (NCs) for brain therapy, no example of PFC nanodroplets which are both stable and small enough to cross the BBB and accumulate in the brain after FUS-BBB opening is known.

Document EP 3095806 A1 relates to specific amphiphilic dendrimers called Dendri-TAC and describes their potential use as theranostic tools. Document US 2021/0252172 A1 relates to an emulsion for use as an improving agent in ultrasound ablation surgery. The article [Calderó, G. et al. Current Opinion in Colloid & Interface Science 49, 118-132 (2020)] is a review covering the themes of ultrasound/radiation-responsive emulsions, emulsion-mediated sonodynamic therapy and ultrasound-propelled nano/micromotors. The article [Astafyeva, K. et al. J. Mater. Chem. B. 3, 2892-2907 (2015)] describes perfluorocarbon nanodroplets stabilized by fluorinated surfactants and their characterization and potentiality as theranostic agents.

### GENERAL PRESENTATION

In what follows, the term "comprise" is synonym of (means the same as) "include" and "contain", is inclusive and open, and does not exclude other non-recited elements. Moreover, in the present disclosure, when referring to a numerical value, the terms "about" and "substantially" are synonyms of (mean the same as) a range comprised between 80% and 120%, preferably between 90% and 110%, of the numerical value.

One of the objectives of the present disclosure is to provide an emulsion allowing BBB transient opening, drug vectorization and subsequent controlled drug delivery, or release, into the brain, in a single and safe formulation.

Such an objective is attained by an emulsion according to a first aspect of the present disclosure. The emulsion according to the first aspect comprises a first discontinuous phase comprising first droplets, a second discontinuous phase comprising second droplets, and a continuous aqueous phase. The first droplets include at least one first surfactant and at least one first fluorocarbon, and have a first mean diameter, D1, higher than a first predetermined value of more than 100 nm. The first droplets are substantially or completely free of any drug. The second droplets include at least one surfactant, at least one drug, and at least one solvent, and have a second mean diameter, D2, less than a second predetermined value of no more than 100 nm.

In the present description and in the claims, by "droplet", it is generally understood a liquid object of substantially spherical shape.

According to a second aspect, the present disclosure concerns an emulsion for use in therapy, e.g., brain therapy, wherein the emulsion is defined as above or according to any of the embodiments described herein. All embodiments directed to the emulsion also apply to the emulsion for a therapeutic use thereof, including the brain therapeutic use.

The inventors have surprisingly found that the emulsion according to the first aspect and embodiments thereof is particularly adapted for brain therapy. The two different populations of droplets present in the emulsion, *i.e.,* the first droplets contained in the first discontinuous phase, and the second droplets contained in the second discontinuous phase, may be tailored to be sensitive to different ultrasound activation thresholds, for example based on selected predetermined values of their diameters D1 and D2. Once administered into the blood vasculature of a brain tissue to be treated by the therapy, the emulsion is ready to undergo an exposure to an external stimulus comprising ultrasounds. Such a stimulus may comprise a series of different US sequences or "insonations". The insonations may be, for example, at least three. Independently of the number of times the external stimulus comprising ultrasounds is applied, the insonation may be characterized by different US parameters, such as e.g., acoustic pressure, frequency, number of cycles, insonation time, duty cycle, pulse duration, pulse repetition frequency (PRF), and playing different roles in the therapy.

For example, a first US sequence followed by a second US sequence may last for an insonation time shorter than the insonation time of the second US sequence and may be made of ultrasonic pulses shorter than the ultrasonic pulses of the second US sequence. Such a first sequence may induce the vaporization of the first droplets into bubbles. A second US sequence lasting for an insonation time longer than the insonation time of the first US sequence but still "short" (for example, less than 500 seconds) and made of pulses longer than the pulses of the first US sequence with a lower acoustic pressure may then induce volume oscillation of the bubbles, thereby allowing localized transient blood-brain barrier BBB opening and penetration of the second droplets into the brain tissue. The second droplets may remain substantially unaffected by the application of the first and second US sequences. Finally, a third optional US sequence may follow the second US sequence. In such a case, the third US sequence may last for an insonation time longer than the insonation time of the second US sequence and may be made of pulses either comparable to or longer than the pulses of the second US sequence. The third US sequence may for example trigger a controlled delivery of a drug contained in the second droplets into a targeted brain tissue.

Hence, one or more embodiments of the emulsion of the present disclosure may allow BBB transient opening, drug vectorization and subsequent controlled drug delivery/release into the brain, while mitigating passive or active release of the drug before BBB-crossing by the second droplets, thereby limiting potential undesired drug side effects. The emulsion of the present disclosure contains the first droplets, which may function as an "opener" and the second droplets, which may function as a "drug-loader", acting in synergy in a single formulation. A mixture of at least two different droplets populations distinguished at least based on the diameter thereof appears well-suited to the constraints of drug delivery in the CNS, while offering sufficient drug accumulation and/or precise spatiotemporal control of the drug delivery into a targeted brain tissue.

Additionally, a predetermined acoustic pressure threshold may be used in each US sequence for achieving the three effects mentioned above, *i.e.,* BBB transient opening, drug vectorization and subsequent optional controlled drug delivery/release into the brain. For example, the acoustic pressure threshold of each first, second and third sequence may be lower than 2MPa, for example as low as 0.2 MPa in one or more embodiments of the present disclosure, thereby avoiding or mitigating biological effects that may be induced by US, such as for example neuroinflammation or other damages to the brain parenchyma.

According to one or more embodiments, the emulsion comprises a dispersion of the first and second discontinuous phases in the continuous aqueous phase. Exemplary characteristics of an emulsion, such as for example the value of the diameter D of the droplets, or the polydispersity index PDI, may change over time. However, according to one or more embodiments of the present disclosure, the emulsion may be in a form that can be used in therapy, *e.g.,* in a form that can be administered into a living human or animal organism. For example, the emulsion according to one or more embodiments of the present disclosure may be administrable, *i.e.,* ready for administration. For example, when the emulsion is intended to be administered by intravenous injection into the body of a patient, the emulsion according to one or more embodiments of the present disclosure may be ready for intravenous injection into the body of a patient. According to one or more embodiments, the emulsion may be administrable as soon as the emulsion is prepared.

According to one or more embodiments, the use in brain therapy comprises a treatment of a disease or disorder affecting a brain or brain tissue targeted by the brain therapy. The disease may for example include a brain disorder, independently of the underlying cause. According to one or more embodiments, the disease is selected from the group comprising neurodegenerative disorders, brain tumors, neuropsychiatric diseases, trauma or epilepsy. The disease or disorder may for example be a brain tumor selected from the group comprising glioblastoma, medulloblastoma, or diffuse intrinsic pontine glioma (DIPG). The disease or disorder may for example be a neurodegenerative disorder selected from the group comprising amyotrophic lateral sclerosis, multiple sclerosis, Alzheimer's disease, or Parkinson's disease.

According to one or more embodiments, the use in brain therapy comprises a controlled release/delivery of the at least one drug contained in the second droplets into a brain tissue, wherein the controlled release/delivery is triggered by an external stimulus comprising ultrasounds. The first droplets do not contain any drug, or are substantially free of any drug.

According to one or more embodiments, the at least one drug contained in the second droplets is selected from the group comprising a drug, a mixture of drugs, a prodrug, a mixture of prodrugs, or combinations thereof. The therapeutic effect sought when using the emulsion in brain therapy may vary depending on the nature of the drug included in the second droplets of the emulsion. According to one or more embodiments, the drug exhibits a biological activity having a beneficial impact on human health. According to one or more embodiments, the at least one drug comprises an anticancer drug, anti-inflammatory drug, antioxidant drug, antibacterial drug, antiviral drug, antifungal drug, antiparasitic drug, antidepressant drug, analgesic drug, anesthetic drug, or a combination thereof. According to one or more embodiments, the at least one drug is selected from the group comprising riluzole, anacardic acid, docetaxel (DTX), paclitaxel (PTX), temozolomide, fluorouracil (5-FU), or Protoporphyrin IX (PP9).

In the therapeutic use of the emulsion as defined in the second aspect of the present disclosure, the drug(s) release into the brain may be controlled by application of an external stimulus, such as a stimulus comprising ultrasounds. Drug release may be controlled in the sense that it may be induced or triggered at a predetermined time, such as, for example, once the second droplets cross the BBB and/or when the second droplets reach at least one location or zone of a brain tissue targeted by the brain therapy, by applying a focused ultrasound beam on the at least one zone of the brain tissue. According to one or more embodiments, the use in brain therapy further comprises monitoring the emulsion by magnetic resonance imaging (MRI) or echography, thus guiding the treatment.

According to one or more embodiments, the therapeutic use of the emulsion comprises administering the emulsion into the blood vasculature of a brain tissue to be treated. Administering the emulsion may comprise administering the emulsion into a living human or animal organism. According to one or more embodiments, administering the emulsion to the living organism is made by intravascular route, e.g., intravenous route, intra-arterial route or a combination thereof. According to one or more embodiments, administering the emulsion is made by intravenous or intra-arterial injection.

According to one or more embodiments, the drug-loaded droplets, *i.e.,* the second droplets of the emulsion according to one or embodiments of the present disclosure, further comprise ligands specific for receptors of the organism in which the emulsion is administered. In this manner, the drug vectorization or drug transport in the body and/or the targeting of brain tissues of interest such as tumors, sites of infection or inflammation, can be improved. According to one or more embodiments, the ligands are hydrophilic. According to one or more embodiments, the ligands comprise arginylglycylaspartic acid (RGD) peptides and/or glycosidic units, such as for example mannose, glucose, or galactose. Such hydrophilic ligands may be placed, substantially, within the at least one second surfactant. For example, the hydrophilic ligands may be placed at an interface of the second discontinuous phase with the continuous aqueous phase.

According to one or more embodiments, the first droplets of the emulsion according to one or embodiments of the present disclosure further comprise ligands specific for certain sites of the BBB, which are placed at an interface of the first discontinuous phase with the continuous aqueous phase.

As mentioned above, the first droplets of the emulsion which may function as opener include at least one first fluorocarbon.

In the present description and in the claims, the term "fluorocarbons" refers to a group of fluorine-containing compounds derived from hydrocarbons by partial or complete substitution of hydrogen atoms with fluorine atoms. According to one or more embodiments, the at least one first fluorocarbon comprises a perfluorocarbon (PFC), *i.e.,* a fluorinated hydrocarbon where all the hydrogen atoms are substituted with fluorine atoms. In the present description and in the claims, a perfluorocarbon is also designated by its acronym PFC.

According to one or more embodiments, the at least one first fluorocarbon comprises a plurality of fluorocarbons, each of which may be defined as above.

According to one or more embodiments, the at least one first fluorocarbon has a boiling point of no more than 100°C. According to one or more embodiments, the at least one first fluorocarbon has a boiling point above 100°C. According to one or more embodiments, the at least one first fluorocarbon has a boiling point above 100°C, and the first droplets further include a second fluorocarbon having a boiling point of no more than 0°C. These embodiments may allow applying acoustic pressures which are sufficiently low in order to safely vaporize the first droplets into bubbles while applying FUS to a targeted brain tissue, when the emulsion is used for brain therapy.

According to one or more embodiments, the at least one first fluorocarbon has a boiling point comprised between -40°C and 200°C, for example comprised between -37°C and 160°C, for example comprised between -2°C and 145°C.

According to one or more embodiments, the at least one first fluorocarbon is selected from the group comprising 1-fluorobutane (boiling point 31.5°C), 2-fluorobutane (boiling point 25.1°C), 2,2-difluorobutane (boiling point 34.4°C), 2,2,3,3-tetrafluorobutane (boiling point 28.6°C), 1,1,1,3,3-pentafluorobutane (boiling point 40.2°C), 1,1,1,4,4,4-hexafluorobutane (boiling point 24-25°C), 1,1,1,2,4,4,4-heptafluorobutane (boiling point 27.5°C), 1,1,2,2,3,3,4,4-octafluorobutane (boiling point 45°C), 1,1,1,2,2,3,4,5,5,5-decafluoropentane (boiling point 53.1°C), 1,1,2,2,3,3,4,4,5,5,6,6-dodecafluorohexane (boiling point 173-174°C), or mixtures thereof.

According to one or more embodiments, the at least one first fluorocarbon is a PFC selected from the group comprising octafluoropropane (boiling point -36.7°C), perfluorobutane (boiling point -1.7°C), perfluoropentane (boiling point 29°C), perfluorohexane (boiling point 57°C), perfluoromethylcyclopentane (boiling point 48°C), perfluoromethylcyclopentane (boiling point 48°C), perfluoro(methylcyclohexane) (boiling point 80-84°C), perfluorooctane (boiling point 99-106°C), perfluorononane (boiling point 123°C), perfluorodecalin (boiling point 140-143°C), perfluorooctylbromide (PFOB) (boiling point 142°C), perfluoro-15-crown-5-ether (PFCE) (boiling point 145°C), or mixtures thereof.

According to one or more embodiments, the first fluorocarbon is a PFC selected from the group comprising perfluoropentane (PFP) (boiling point 29°C), perfluorohexane (PFH) (boiling point 57°C), or a mixture thereof.

According to one or more embodiments, the first droplets and second droplets are dispersed in the continuous aqueous phase of the emulsion. The at least one first and the at least one second surfactant may comprise amphiphilic compounds which may encapsulate the first and second droplets, respectively, while imparting stability thereto.

According to one or more embodiments described hereinafter, the stability of the first and second droplets may be further improved by selecting the at least one first surfactant and the at least one second surfactant from compounds that are suitable for imparting stability and/or other properties to the first and second droplets.

According to one or more embodiments, the at least one first surfactant and/or the at least one second surfactant is/are selected from the group comprising dendrimers of Dendri-TAC type, oligomers of F*ᵢ*TAC*ₙ* or H*ᵢ*TAC*ₙ* type, TPGS 1000, TPGS 750M, surfactants derived from sugars and/or amino acids, and combinations thereof.

The at least one first and/or second surfactant may be anionic, cationic, zwitterionic, amphoteric or nonionic. They may be natural or synthetic. According to one or more embodiments, their hydrophilic-lipophilic balance (HLB) is comprised between 4 and 19. The at least one first and/or second surfactant may, for example, be selected from the group comprising nonionic surfactants such as alcohols, amino alcohols, esters, amine oxides, and combinations thereof. The at least one first and/or second surfactant may be, for example, Lauroglycol^{™} FCC (Propylene glycol monolaurate (type I)), Lauroglycol^{™} 90, Capryol^{™} 90, Plurol^{®} Oleique CC 497 (Polyglyceryl-3 dioleate), Labrasol^{®}, Caprylocaproyl Polyoxyl-8 glycerides, Labrafil^{®} M 1944 CS, Labrasol^{®} ALF, Labrafil^{®} M 2125 CS, Gelucire^{®} 50/13, Labrafil^{®} M 2130 CS, Gelucire^{®} 48/16, Gelucire^{®} 44/14, Transcutol^{®} HP, Emulfree^{®} CBG MB. The at least one first and/or second surfactant may be selected from the group consisting of Axol C62 (ADARA), Cutina CP (BASF), Cutina GMS (BASF), Emulium 22 (GATTEFOSSE), Emulium Delta (GATTEFOSSE), Emulium melifera (GATTEFOSSE), Eumulgin SG (BASF), Montanov 202 (Seppic), Montanov 68 (Seppic), Montanov 82 (Seppic), Montanov L (Seppic), Olivem 1000 (Univar), Olivoil avenate (ACS Phyto), Xyliance (MASSO). The at least one first and/or second surfactant may be derived from sugars, such as an alkyl polyglycoside, a sorbitan ester, a sucrose ester (or sucroester), or an alkylmethylglucamide. For example, the at least one first and/or second surfactant may be may be an alkyl polyglycoside, such as an alkyl polyglucoside. The at least one first and/or second surfactant may be a fatty acid sucroester, or sucrose ester of fatty acid, such as for example E473. The at least one first and/or second surfactant may be a sorbitan ester, such as E491 (Sorbitan monostearate, Span 60), E492 (Sorbitan tristearate, Span 65), E493 (Sorbitan monolaurate, Span 20), E494 (Sorbitan monooleate, Span 80), E495 (Sorbitan monopalmitate, Span 40), E496 (Sorbitan trioleate, Span 85), E432 (Polyoxyethylene sorbitan monolaurate, Polysorbate 20), E433 (Polyoxyethylene sorbitan monooleate, Polysorbate 80), E434 (Polyoxyethylene sorbitan monopalmitate, Polysorbate 40), E435 (Polyoxyethylene sorbitan monostearate, Polysorbate 60), E436 (Polyoxyethylene sorbitan tristearate, Polysorbate 65). The at least one first and/or second surfactant may be selected from the group consisting of Simulsol AS 48, Simulsol SL 7G, Coco Glucoside, Decyl Glucoside, Lauryl Glucoside, Sodium Lauryl Glucose Carboxylate.

The at least one first and/or second surfactant may be derived from amino acids, such as Sodium Cocoyl Glutamate, Disodium Cocoyl Glutamate, Sodium Lauroyl Glutamate, derived from peptides, such as Sodium Cocoyl Hydrolyzed Wheat Protein, Sodium Cocoyl Hydrolyzed Collagen. The at least one first and/or second surfactant may be an alkyl PEG sulfosuccinate such as Disodium Laureth Sulfosuccinate, Disodium Deceth Sulfosuccinate, an acylsarcosine such as Sodium Lauroyl Sarcosinate, Sodium Cocoyl Sarcosinate, an acyl isethionate such as Sodium Cocoyl Isethionate. The at least one first and/or second surfactant may be Disodium Laureth Sulfosuccinate, Cocamidopropyl Betaine, Coco Betaine, Sodium Coco Sulfate, Sodium Lauryl Sulfoacetate. The at least one first and/or second surfactant may be a glycolipid such as rhamnolipids (RL), mannosylerythritol lipids (MEL), trehalipids (TL), xylolipids, sophorolipids, a lipopeptide such as fengycin, iturin, surfactin.

According to one or more embodiments, the at least one first surfactant and/or the at least one second surfactant is/are selected from the group comprising a dendrimer of Dendri-TAC type, an oligomer of F*ᵢ*TAC*ₙ* or H*ᵢ*TAC*ₙ* type, or a mixture thereof. For example, according to one or more embodiments, the at least one first surfactant comprises an oligomer of F*ᵢ*TAC*ₙ* type. According to one or more embodiments, the at least one second surfactant comprises a dendrimer of Dendri-TAC type.

According to one or more embodiments, the at least one first surfactant and/or the at least one second surfactant comprises/comprise a dendrimer of Dendri-TAC type, such as those described in PCT application WO 2016/185425 A1. Dendri-TACs constitute a class of amphiphilic compounds which may have self-assembly properties and may be highly modular, either in terms of the hydrophobic tail or in terms of the multiplication of the branches of the hydrophilic head.

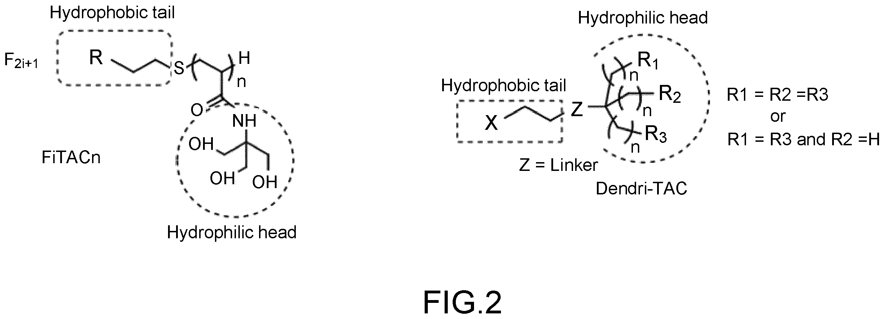

Exemplary Dendri-TAC surfactants are shown in FIG. 2, right-hand part. These exemplary Dendri-TAC surfactants offer many opportunities for drug encapsulation, due to their dendritic molecular architecture allowing the appearance of internal cavities that can constitute as many compartments in which a drug can be stored before release. According to one or more embodiments, the hydrophobic tail of the Dendri-TAC surfactant is selected from the group comprising a branched or linear fluoroalkyl chain, a branched or linear alkyl chain.

According to one or more embodiments, the at least one first surfactant comprises an oligomer of F*ᵢ*TAC*ₙ* type. According to one or more embodiments, the at least one second surfactant comprises an oligomer of F*ᵢ*TAC*ₙ* or H*ᵢ*TAC*ₙ* type. The amphiphilic compounds of F*ᵢ*TAC*ₙ* or H*ᵢ*TAC*ₙ* type may not only impart improved stability to the first and/or second droplets, but also exhibit good biocompatibility.

Exemplary F*ᵢ*TAC*ₙ* surfactants are shown in FIG. 2, left-hand part. These exemplary F*ᵢ*TAC*ₙ* surfactants include a hydrophilic head (or "polar head") comprising an oligomer of polyTRIS type, and a hydrophobic tail comprising a fluoroalkyl linear chain. In the case of H*ᵢ*TAC*ₙ* surfactants, the fluorine atoms are replaced by hydrogen atoms in the hydrophobic tail. Thus, *n* is the degree of oligomerization of the polyTRIS portion and *i* is the number of carbon atoms of the hydrophobic tail. According to one or more embodiments, i is between 6 and 10. According to one or more embodiments, when *i* is between 6 and 10, *n* is between 1 and 40, for example between 4 and 30. According to one or more embodiments, when i is 8, n is between 1 and 40, for example between 4 and 30.

According to one or more embodiments, the at least one first surfactant and/or the at least one second surfactant is/are selected from the group comprising F₆TAC₇, F₆TAC₁₂, F₆TAC₂₉, F₈TAC₇, F₈TAC₁₃, F₈TAC₁₇, or F₈TAC₁₈. According to one or more embodiments, the first or second surfactant comprises F₈TAC₁₃.

According to one or more embodiments of the present disclosure, the first droplets have a diameter D1 of more than 100 nm, and the second droplets have a diameter D2 of no more than 100 nm. In brain therapy, when the emulsion is activated by ultrasounds and the BBB is transiently open or disrupted, this exemplary size or another predetermined size of the first droplets may prevent the first droplets from crossing the BBB, while this exemplary size or another predetermined size of the second droplets may allow the second droplets to cross the BBB.

The first mean diameter D1 of the first droplets is the mean diameter of the population of first droplets. Likewise, the second mean diameter D2 of the second droplets is the mean diameter of the population of second droplets. According to one or more embodiments, the first diameter D1 of the first droplets and the second mean diameter D2 of the second droplets are measured by dynamic light scattering (DLS). Details regarding such a DLS measurement are provided in part IV of the detailed description. Each of the first mean diameter of the first droplets and the second mean diameter of the second droplets may be an intensity-weighted mean diameter derived from the cumulants analysis, or from Contin analysis. In other words, the diameter D1 and/or D2 may be the average size of calibrated nanodroplets dispersed in an aqueous suspension, measured through the dynamic light scattering technique (DLS). In certain cases, for example in case of spectroscopic incompatibility inherent to the composition of the droplets *(i.e.,* in case of overlap between the absorbance of the droplets and the wavelength of the light emitted by the DLS apparatus), Transmission Electron Microscopy (TEM), or cryo-TEM may also be used to measure D1 and/or D2.

Unless otherwise indicated, in the present description and in the claims, the diameter and the polydispersity index PDI herein referred to correspond to the diameter and the PDI, respectively, of the emulsion in an administrable form, as defined above.

In the present description and in the claims, the term "polydispersity index" (PDI) is a dimensionless parameter representative of the broadness of the size distribution calculated from the cumulants analysis, or from Contin analysis. The cumulants analysis, defined in the International Standard on Dynamic Light Scattering ISO13321 (1996) and ISO22412 (2008), gives a mean particle size (z-average) and an estimate of the width of the distribution (polydispersity index).

For instance, a polydispersity higher than 0.7 indicates a broad distribution of particles sizes, while a value lower than 0.08 indicates a nearly monodisperse sample having a monomodal distribution. The polydispersity can be measured with the dynamic light scattering technique (DLS), by using for instance the Malvern Zetasizer Nano-ZS instrument (Malvern Instruments Ltd., UK).

According to one or more embodiments, the first mean diameter is comprised between 200 nm and 600 nm and/or the second mean diameter is comprised between 20 nm and 95 nm.

According to one or more embodiments, the first mean diameter is comprised between more than 100 nm and 600 nm. The bubbles resulting from the vaporization of the first droplets, *i.e.,* from the vaporization of the at least one fluorocarbon, may have a diameter which may reach 5 times the first mean diameter. Accordingly, an upper limit of 600 nm for the first mean diameter allows increasing the safety associated to the use of the emulsion in brain therapy, considering that microbubbles with a diameter of *e.g.,* 10 µm may occlude a blood vessel of the blood vasculature of a patient. According to one or more embodiments, the first mean diameter is comprised between 150 nm and 600 nm, *e.g.,* between 200 nm and 500 nm, *e.g.,* between 250 nm and 450 nm, *e.g.,* between 300 nm and 400 nm. According to one or more embodiments, the first mean diameter is comprised between more than 400 nm and 600 nm. According to one or more embodiments, the first mean diameter is comprised between more than 100 nm and 300 nm, e.g., between more than 100 nm and 200 nm.

According to one or more embodiments, the second mean diameter is comprised between 20 nm and 95 nm, *e.g.,* between 25 nm and 85 nm, *e.g.,* between 35 nm and 75 nm.

According to one or more embodiments, the first droplets have a first PDI of no more than 0.5, for example lower than 0.25, lower than 0.2, lower than 0.1. According to one or more embodiments, which may be combined with any of these exemplary values of the first PDI, the second droplets may have a second PDI of no more than 0.5, for example lower than 0.25, lower than 0.2, lower than 0.1.

The second droplets of the emulsion according to the present disclosure include at least one solvent. According to one or more embodiments, the at least one solvent is selected from the group comprising lipophilic compounds, fluorocarbons, or mixtures thereof. For example, the at least one solvent can comprise a fluorocarbon, or a mixture of different fluorocarbons.

According to one or more embodiments, the at least one solvent comprises at least one PFC selected from the group comprising perfluorooctane, perfluorononane, perfluorodecalin, perfluorooctyl bromide (PFOB), perfluoro-15-crown-5-ether (PFCE), 1,1,1-tris(perfluorotert-butoxymethyl)ethane (TPFBME), or mixtures thereof. For example, according to one or more embodiments, the at least one solvent comprises substantially PFOB, or consists of PFOB. Such a PFC exhibits both a low toxicity and an acceptable excretion profile, *i.e.,* an organ retention half-life of 3-4 days in humans.

According to one or more embodiments, the at least one solvent comprises a lipophilic compound selected from the group comprising mono-, di-, or tri-esters of glycerol or derivatives thereof, mono-, di-, tri- or tetra-esters of citric acid or derivatives thereof, fatty acids, monoesters of fatty acids, sterol esters, sphingolipids, glycerophospholipids, polyketides, saccharolipids, terpenes, lipid derivatives of prenol, or mixtures thereof. According to one or more embodiments, the lipophilic compound is selected from the group comprising tributyl O-acetyl citrate (ATBC), Capryol 90 oil (C90), tripropionin, tributyrin, soybean oil, or a mixture thereof. According to one or more embodiments, the lipophilic compound is selected from the group comprising propylene glycol mono-caprylate, propylene glycol di-caprylate, or a mixture thereof.

For example, according to one or more embodiments, the at least one solvent comprises a mixture of a fluorocarbon with a lipophilic compound, such as for example PFOB with ATBC, or PFOB with C90.

In certain embodiments, the at least one solvent may not solubilize the at least one drug. For example, the at least one drug may be hydrophilic and "stored" within the hydrophilic head of the at least one second surfactant, for example *via* non-covalent interactions between the hydrophilic head and the at least one drug.

According to one or more embodiments, the emulsion further comprises at least one third discontinuous phase comprising third droplets. The third droplets may include at least one third surfactant, at least one solvent and at least one drug which may be equal to or different from the drug contained in the second droplets. Apart from the latter possible difference, the third droplets may be identical or very similar to the second droplets, in the sense that the third droplets may have a third diameter D3 having a third predetermined value, which may be of no more than 100 nm, and may be measured as described above with reference to D1 and D2. In this case, the role of the third droplets of the emulsion used in brain therapy may be the same as the role of the second droplets as defined above.

According to one or more embodiments, at least the at least one first surfactant, the at least one second surfactant, the at least one fluorocarbon, and the solvent are biocompatible. "Biocompatible" intends to indicate a substance without toxic or harmful effects on health, and which can be used, for example, in pharmaceutical applications. For example, according to one or more embodiments, at least the above substances can be considered harmless according to the criteria recognized by the USFDA (United States Food and Drug Administration) and may be classified as "GRAS" (for "Generally Recognized As Safe").

According to one or more embodiments, the emulsion comprises, in the continuous aqueous phase and/or the first discontinuous phase and/or the second discontinuous phase, at least one excipient. According to one or more embodiments, the at least one excipient is selected from the group comprising acidifying agents, basifying agents, buffering agents, or stabilizers, such as, for example, cryoprotectants, lyoprotectors. According to one or more embodiments, the at least one excipient is present predominantly or substantially in the aqueous phase of the emulsion.

According to one or more embodiments, the continuous aqueous phase comprises at least one salt. According to one or more embodiments, the at least one salt is selected from the group comprising NaCl. According to one or more embodiments, the salt concentration in the continuous aqueous phase is comprised between 0.6 and 1.2 % w/v, for example 0.9 % w/v.

According to a third aspect, the present disclosure concerns a dry formulation obtained by drying the emulsion as defined in any one of the embodiments according to the first aspect.

According to one or more embodiments, the dry formulation is substantially free of water. According to one or more embodiments, the water content of the dry formulation is less than 10 wt% relative to the total weight of the dry formulation, for example less than 5 wt%, 4 wt%, 3 wt%, 2 wt%, 1 wt%.

According to a fourth aspect, the present disclosure concerns a dry formulation for use in brain therapy, wherein the dry formulation is defined in any of the embodiments described herein. All embodiments directed to the dry formulation also apply to the dry formulation for a therapeutic use thereof, including the brain therapeutic use.

The dry formulation is obtained by drying the emulsion. According to one or more embodiments, the drying of the emulsion comprises spray-drying, freeze-drying, electrospinning, or combinations thereof. According to one or more embodiments, drying the emulsion includes freeze-drying the emulsion.

According to one or more embodiments, the dry formulation further comprises at least one cryoprotectant selected from the group comprising polymers, amino acids, saccharide compounds such as mono-, di- and polysaccharides, or combinations thereof. According to one or more embodiments, the at least one cryoprotectant is selected from the group comprising trehalose, sucrose, maltose, glucose, mannitol, hydroxypropyl-β-cyclodextrin, and combinations thereof. In other exemplary embodiments, the at least one cryoprotectant is a polymer, such as a polyvinylpyrrolidone or a polyvinyl alcohol. The at least one cryoprotectant can also be an amino acid, such as glycine.

The drying can for example be adapted to the nature of the drug and its inherent sensitivity. The duration of the drying can vary from a few hours to several days.

According to one or more embodiments, when the drying includes a freeze-drying step, the freeze-drying step can include at least three sub-steps: freezing; sublimation to sublimate water from said mixture; and desiccation to reduce the residual moisture content. According to one or more embodiments, in freezing, the emulsion is brought to a temperature below a predetermined temperature, for example below 0°C, below -10°C or below, such as at about -20°C, or about -40°C. According to one or more embodiments, the sublimation may be performed at a pressure lower than a predetermined pressure, for example between 50 and 1000 µbars, for example at about 500 µbars. According to one or more embodiments, in the desiccation said resulting mixture is brought to a temperature above a predetermined temperature, for example above about 10°C, for example above about 20°C.

From the dry formulations according to the third aspect, the corresponding emulsions may be easily reconstituted by simple rehydration, *i.e.,* by adding a certain amount of water or aqueous solution to the dry formulation. The emulsion thus reconstituted by rehydration may not necessarily have the same characteristics as the starting emulsion, *i.e.,* the emulsion before drying. For example, the first and second mean diameter of the first and second droplets, respectively, and/or the corresponding first and second polydispersity indexes PDI of the reconstituted emulsion may have values differing from their values in the emulsion before drying.

The therapeutic use of the dry formulation may include administering the dry formulation into a target organism. According to one or more embodiments, the administration of the dry formulation into the target organism of a brain therapy is made by intravascular route. According to one or more embodiments, the dry formulation also allows long-term storage prior to use in liquid form, *e.g.,* for intravascular administration, after addition of water or an aqueous solution to the dry formulation.

According to a further aspect, the present disclosure concerns a therapeutic method. The method may include a method for delivering a drug into a brain tissue. The therapeutic method comprises the following steps:
- administering the emulsion as defined in any one of the embodiments described herein into the blood vasculature of a brain tissue to be treated;
- applying a first focused ultrasound beam for a predetermined insonation time to at least one zone of the brain tissue;
- applying a second focused ultrasound beam for a predetermined insonation time to the at least one zone of the brain tissue;
- applying a third focused ultrasound beam to the at least one zone of the brain tissue.

Applying the focused ultrasound beam may induce vaporization of the first droplets into bubbles. Applying the second focused ultrasound beam may induce volume oscillation of the bubbles, and thus allow localized transient brain blood barrier BBB opening and penetration of the second droplets into the brain tissue. Applying the third focused ultrasound beam may trigger the controlled delivery of the drug contained in the second droplets into the brain tissue.

According to one or more embodiments, the administering of the emulsion may be as defined above with respect to any one of the embodiments of the therapeutic use of the emulsion (e.g., by intravenous or intra-arterial injection, or retro-orbital injection). Likewise, the exemplary US parameters described below with respect to the therapeutic method, such as, e.g., acoustic pressure, insonation time, may also apply to the therapeutic use as defined above with respect to the emulsion and to the dry formulation according to the present disclosure.

According to one or more embodiments, the first focused ultrasound beam may have an acoustic pressure of no more than 2,5 MPa, for example of no more than 2 MPa. According to one or more embodiments, the first focused ultrasound beam is applied in pulses for a predetermined insonation time comprised between 1 µs and 1 ms, for example comprised between 1 µs and 100 µs. The first focused ultrasound beam may for example be applied in pulses having a pulse duration of less than 100 µs, e.g., comprised between 10 µs and 50 µs. The pulses of the first focused ultrasound beam may for example comprise between 1 and 20 cycles, for example between 1 and 10 cycles. The first focused ultrasound beam may be characterized by a frequency comprised between 400 kHz and 2 MHz, and/or by a pulse repetition frequency (PRF) comprised between 0 Hz *(i.e.,* a single pulse is sent and the second focused ultrasound beam is used right after, in order to avoid droplet recondensation) and 10 kHz.

According to one or more embodiments, the acoustic pressure of the second focused ultrasound beam is of no more than 1.5 MPa, for example of no more than 1 MPa, for example comprised between 0.2 MPa and 0.8 MPa. According to one or more embodiments, the second focused ultrasound beam is applied in pulses for a predetermined insonation time comprised between 10 s and 500 s, for example comprised between 30 s and 400 s. The second focused ultrasound beam may for example be applied in pulses having a pulse duration of less than 50 ms, e.g., comprised between 2 ms and 30 ms. The pulses of the second focused ultrasound beam may for example comprise between 1000 and 100000 cycles, for example about 10000 cycles. The second focused ultrasound beam may be characterized by a frequency comprised between 400 Hz and 2 MHz, and/or by a pulse repetition frequency (PRF) comprised between 1 Hz and 5 Hz.

According to one or more embodiments, the acoustic pressure of the third focused ultrasound beam is of no more than 1.5 MPa, for example of no more than 1 MPa, for example comprised between 0.1 MPa and 1 MPa. According to one or more embodiments, the third focused ultrasound beam is applied in pulses for a predetermined insonation time of above 1 mn, for example of above 5 mn, comprised between 5 mn and 10 mn. The third focused ultrasound beam may for example be applied in pulses having a pulse duration of less than 10 ms, e.g., comprised between 50 µs and 1 ms. The pulses of the third focused ultrasound beam may for example comprise between 50 and 10000 cycles, for example about 400 cycles. The third focused ultrasound beam may be characterized by a frequency comprised between 0.4 Hz and 2 MHz, of for example about 800 Hz, and/or by a pulse repetition frequency (PRF) comprised between 100 Hz and 800 Hz.

Other features and advantages of the invention will become apparent from the following detailed description, taken in conjunction with the accompanying drawings, which illustrate, by way of example, the principles of the invention.

### BRIEF DESCRIPTION OF THE DRAWINGS

In the drawings, like reference signs generally refer to the same or like parts throughout the different views. The drawings are not necessarily to scale, emphasis instead generally being placed upon illustrating the principles of the invention.
FIG. 1 is a schematic representation of an example of therapeutic use of an emulsion according to embodiments of the present description.
FIG. 2 is a representation of a molecular structure of a first and of a second example of surfactants that may be used in emulsions or dry formulations according to embodiments of the present description.
FIG. 3 shows two pictures of model second droplets that may be used in emulsions or dry formulations according to embodiments of the present description (left: TEM image; right: cryo-TEM).
FIG. 4 is a histogram representation of drug concentration and encapsulation efficiency depending on the lipophilic compound used in the solvent of example of second droplets.
FIG. 5 is a graphical representation of the variation with the D1 diameter of the peak-to-peak pressure at which the ADV probability is 0.5, for example of first droplets comprising PFH.

### DETAILED DESCRIPTION OF EXAMPLES

Examples of emulsions and dry formulations will now be described in detail with reference to the accompanying figures. In the following detailed description, numerous specific details are set forth in order to provide a more thorough understanding of the invention. However, it will be apparent to one of ordinary skill in the art that the invention may be practiced without these specific details. In other instances, well-known features have not been described in detail to avoid unnecessarily complicating the description.

The following description provides non-limiting examples of emulsions and dry formulations according to the present disclosure. The description further provides non-limiting details about the stability of emulsions according to embodiments of the present disclosure, as well as details concerning exemplary embodiments of therapeutic uses of emulsions in brain therapy.

### I. Illustration of an emulsion for use in brain therapy according to one or more embodiments

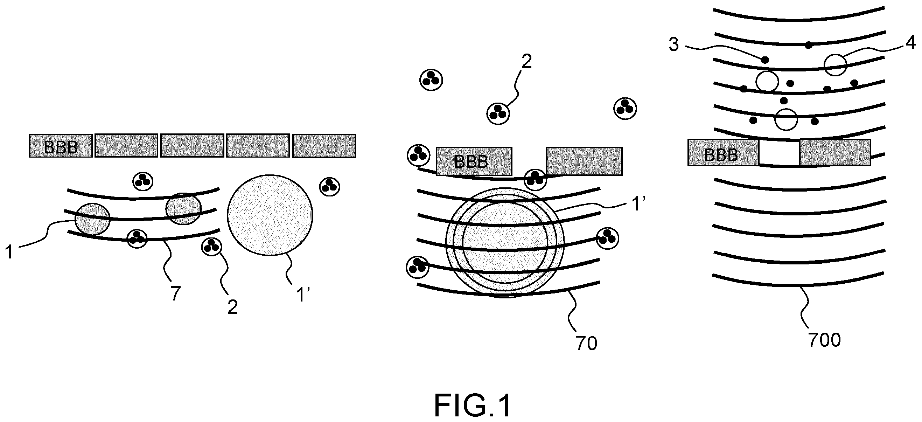

FIG. 1 illustrates an example of an emulsion for therapeutic use including controlled drug release in the brain according to embodiments of the present disclosure.

The therapeutic use illustrated in FIG. 1 comprises the administration of an emulsion into an organism, with a view to targeting a brain tissue comprised in the brain parenchyma of said organism. The brain tissue may for example be affected by a disorder or a disease. The disease may for example be selected from the group comprising neurodegenerative disorders, brain tumors, neuropsychiatric diseases, trauma, vascular injury or epilepsy.

The emulsion includes a first discontinuous phase comprising first droplets 1, a second discontinuous phase comprising second droplets 2, and a continuous aqueous phase. The first droplets 1 include at least one first surfactant and at least one first fluorocarbon, and have a first mean diameter of more than 100 nm. The second droplets 2 include at least one surfactant, at least one drug 3, and at least one solvent, and have a diameter of no more than 100 nm. The at least one drug 3 may be specific to the disease or disorder affecting the brain tissue.

Once administered into the organism, the emulsion may reach the blood vasculature of the brain tissue to be treated. The emulsion may then be ready to undergo an exposure to an external stimulus comprising ultrasounds. Such a stimulus may consist in a series of, for example, at least three different US sequences or "insonations", such as depicted in FIG. 1.

Three different US sequences (7, 70, 700) are indeed represented in FIG. 1, and may be characterized by different US parameters, such as e.g., acoustic pressure, frequency, number of cycles, insonation time, duty cycle, pulse duration, pulse repetition frequency (PRF), as each US sequence may play a different role in brain therapy.

For example, a first US sequence (or US beam) 7 lasting a very short insonation time, *e.g.,* comprised between 1 µs and 1 ms, and made of short ultrasonic pulses *e.g.,* comprised between 10 µs and 50 µs, may induce the vaporization of the first droplets 1 into bubbles 1' (See FIG. 1, left).

The acoustic pressure of the first focused US beam may for example be of no more than 2,5 MPa, for example of no more than 2 MPa.

Then, a second US sequence (or US beam) 70 lasting a short insonation time, for example comprised between 10 s and 500 s, and made of longer pulses, e.g., comprised between 2 ms and 30 ms, with a lower acoustic pressure, for example of no more than 1.5 MPa, for example of no more than 1 MPa, may then induce volume oscillation of the bubbles 1'. Such an oscillation may allow localized transient blood-brain barrier BBB opening and penetration of the second droplets 2 into the brain tissue (See FIG. 1, middle). The second droplets 2 may remain substantially unaffected by the application of these two first and second US sequences 7, 70.

Finally, a third US sequence 700 lasting a long insonation time, for example comprised between 5 min and 45 min, and made of repeated long pulses e.g., comprised between 5 ms and 20 ms, may trigger the controlled release of the drug 3 contained in the second droplets 2 into the targeted brain tissue (See FIG. 1, right).

Without wishing to be bound by any theory, the inventors believe that applying an external stimulus, e.g., ultrasound, in order to trigger the controlled release of the drug 3, may have the effect of increasing the permeability of the "shell" of the droplets 2, namely the part of the droplets located substantially at the surface of the droplets. This increase in permeability can for example be caused by an acoustic radiation force when the droplets 2 are subjected to ultrasound, and facilitates the diffusion of the drug 3 out of the droplets during the application of the external stimulus, thereby releasing the drug 3 and leaving a droplet 4 fully or partly emptied of the drug 3. According to one or more embodiments, the controlled drug release is not triggered by cavitation and/or vaporization of a compound present in the droplets, but by such an US-triggered increase in permeability of the droplets 2.

Hence, the drug release into the brain may be controlled, in the sense that the drug release may be induced or triggered at a predetermined time, such as once the second droplets cross the BBB and/or when the second droplets reach at least one location or zone of a brain tissue targeted by the brain therapy, by applying a focused ultrasound beam on said at least one zone of the brain tissue.

However, according to one or more embodiments of emulsions for therapeutic use according to the present description, other predetermined times can be envisaged, e.g., a time when a certain concentration of droplets 2 is detected in the vicinity of a brain tissue targeted by the therapy.

### II. Illustrative preparation of an example of emulsion according to one or more embodiments

This part II describes first of all examples of separate preparations of emulsions and dry formulations containing either "first droplets" or "second droplets", said droplets being as defined in one or more embodiments of the emulsion and dry formulation according to the present disclosure (sections a. and b.). This part II further describes examples of preparation of emulsions according to the present disclosure, *i.e.,* combining both types of droplets, *i.e.,* the first droplets *and the* second droplets, and the observation of their stability in the presence of human plasma (section c.).

### a. Preparation of emulsions containing "first droplets" (ND1 and ND1-FITC)

Emulsions ND1 were made of perfluorohexane (PFH) droplets stabilized by the fluorinated surfactant F₈TAC₁₃. The emulsions ND1-FITC were prepared following the same procedure as for ND1, except that a fluorinated derivative of fluorescein isothiocyanate (FITC, a fluorescent tag), *i.e.,* F₆H₂-FITC (synthesis described below) was added to the droplet shell.

4.88 mg of F₈TAC₁₃ was dissolved in 4.88 mL of water to prepare ND1 emulsion, while 4.83 mg of F₈TAC₁₃ and 0.0488 mg of F₆H₂-FITC were dissolved in 4.88 mL of water to prepare ND1-FITC emulsion. 120 µL of PFH were added to each of these solutions.

The resulting mixtures were vortexed to create coarse emulsions. A high-pressure microfluidizer (model LV1 from Microfluidics^{®}) was used to reduce the size of the droplets. The mixture was passed through the microfluidizer 8 times. Then, the emulsions were separated into different 2 mL centrifuge tubes (1mL of mixture per tube), which were then centrifugated 30s at room temperature at 2000 g. The supernatant was kept while the pellet was removed, allowing to remove the bigger size droplets while keeping the smaller sizes. A second centrifugation step was carried out at 6000 g for 40 min at 4°C. The supernatant was discarded while the pellet was kept and re-suspended at the same concentration, allowing to remove the very small droplets, thereby creating a more homogeneous population. The diameter D1 of the droplets was more than 100 nm, and comprised between 200 nm and 600 nm, as measured by DLS (cumulants analysis).

### Preparation of dry formulations of ND1 and ND1-FITC

The emulsions ND1 and ND1-FITC were freeze-dried in 2 mL centrifuge tubes after having added 50 mg of trehalose. The tubes (aliquots of 1 mL) were freeze-dried overnight using a Labconco^{®} freeze drier at a temperature lower than -50°C and at a pressure lower than 100 Pa. Two types of dry samples of ND1 and NDI-FITC were thus obtained, containing 3.2 x 10¹¹ and 7 x 10¹¹ droplets, respectively, as estimated from the weight of the dry samples and the diameter measured by DLS.

Preparation of F₆H₂-FITC: FITC (105 mg, 0.27 mmol) was dried under high vacuum for 5 min and then covered with a blanket of nitrogen. 1H,1H,2H,2H-Perfluorooctan-1-ol (1.5 ml, 6.9 mmol) was added and the mixture was heated at 50°C for 72 hours. The solvent was then removed under high vacuum. The crude was purified by flash chromatography to obtain 80 mg of pure compound with a yield of 39% (ethyl acetate/cyclohexane 5/5 with 0.5 % formic acid, Rf= 0.56). ¹H NMR (MeOD, 400 MHz) δ 8.48 (1H, s), 7.97 (1H, s), 7.17 (1H, d, J =8.19Hz), 6.68 (2H, s, J =2.31Hz), 6.62 (2H, d, J =8.70Hz), 6.54 (2H, dd, J =8.70Hz, J =2.31Hz), 4.90 (2H, m), 2.80 (2H, t, J =19.38Hz); ¹³C NMR (MeOD, 100 MHz) δ 189.4, 171.0, 161.5, 154.2, 130.2, 125.7, 119.0, 113.7, 111.4, 103.6, 62.5, 31.3; ¹⁹F NMR (MeOD, 100 MHz) -82.4 (3H, m), -114.1 (2H, br), -122.8 (2H, s), -123.9 (2H, s), -124. 6 (2H, s), -127.3 (2H, br).

### b. Preparation of emulsions containing "second droplets" (ND2-DiD, ND2-PTX, ND2-DTX)

### Preparation of emulsion ND2-DiD

In the following preparation of emulsion ND2-DID, "DiD" is a fluorescent tag, *i.e.,* (DiIC₁₈(5)-DS(1,1'-Dioctadecyl-3,3,3',3'-Tetramethylindodicarbocyanine, 4-Chlorobenzenesulfonate salt)) which models the at least one drug included in the second droplets.

The surfactant F₈TAC₁₃ (53 mg) was dissolved in water (2 mL). A fresh solution of DiD (0.5 wt%) in tributyl O-acetyl citrate (ATBC) was prepared with a US bath and a vortex mixer. The resulting oil (5 µL) was introduced in a 50 mL centrifuge tube. PFOB (95 µL) was added, followed by the aqueous solution containing the surfactant. The mixture was cooled to 0 °C with an ice bath, and was then insonated for 2 min in pulse mode (Duty Cycle 11.97%, pulse width 8 s) with a sonicator (VibraCell. TM. 75043, 750 W, Bioblock Scientific, USA), using a 13 mm sonotrode, placed at the bottom of the centrifuge tube, at an amplitude of 60%. The resulting emulsion was centrifuged for 5 min at 5580 g. The supernatant was collected and centrifuged for 20 min at 25830 g. The supernatant was then removed (1.5 mL) and the cake rinsed twice with water (1.5 mL). The cake was then resuspended in water (1.5 mL) with a vortex mixer. This workup was repeated once to obtain a ND2-DID emulsion.

### Preparation of a dry formulation of ND2-DiD

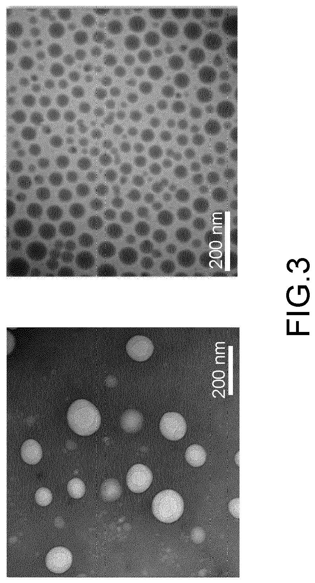

In 2 mL vials, an aliquot (300 µL) of emulsion ND2-DiD was added along with 45 µL trehalose solution (in water; [C] = 357 mg mL⁻¹). After being frozen at -20°C for 4 hours, the vials were freeze-dried overnight. Each vial was kept at 4°C until use. Each vial was then reconstituted by adding the 300 µL water extemporaneously. The volume fraction in PFOB (measured by ¹⁹F-NMR) of the reconstituted emulsion was 2.57%. The diameter D2 of resuspended droplets was 75±24 nm, as measured by TEM (see FIG. 3, left), corresponding to a total of 3.49.10¹³ droplets per reconstituted sample of 300 µL, as estimated from the volume fraction in PFOB and the diameter measured by TEM. Cryo-TEM was also used to measure the diameter D2, providing a D2 value of 43±13 nm (FIG. 3, right). Due to an overlapping between the absorbance of DiD and the emitted light of the DLS apparatus (operating at 633 nm), the detection of the scattered light is not suitable for DiD-tagged droplets. Accordingly, D2 was assessed by TEM or cryo-TEM in this case.

### Preparation of emulsions ND2-PTX and ND2-DTX

To two 2 mL vials, docetaxel (DTX) was added in excess to a lipophilic compound: 39 mg of DTX in 300 µl ATBC, 30 mg of DTX in 250 µl C-90. The resulting suspensions were agitated with a magnetic stirrer for 2 hours. The vials were centrifuged at 5580 g for 10 min, and the supernatant was titrated by RP-HPLC (sample diluted with ACN). A PTX-loaded oil was prepared in the same manner, using the C-90 oil.

The three resulting oils loaded with PTX or DTX were used in the following preparation of ND2-PTX and ND2-DTX emulsions. In these examples, the emulsion composition is as follows: surfactant F₈TAC₁₃ (380 mg/ml of solvent, corresponding to 155 mg of surfactant for 0.4 ml of solvent), water 2 mL, solvent (95/5 PFOB/oil % v/v) 0.4 mL.

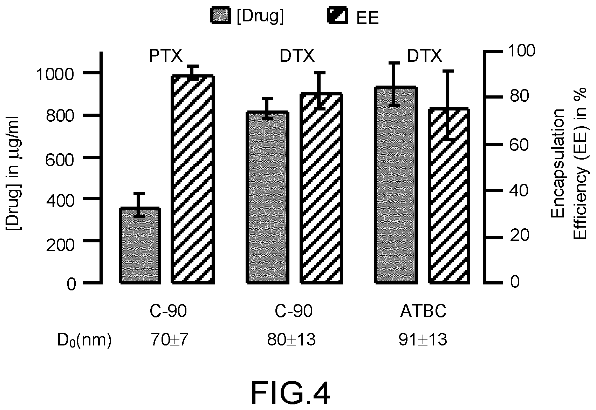

Under these emulsification conditions, an increase in drug concentration was observed when comparing PTX with DTX, rising from 370 ± 58 mg/ml of emulsion for PTX-loaded C-90 oil, to 830 ± 44 mg/ml of emulsion for DTX-loaded C-90 oil, and 946 ± 100 mg/ml of emulsion for DTX-loaded ATBC oil (See Scheme 3). The droplets' diameters D2 of the three emulsions were 70 nm, 80 nm and 91 nm, respectively, as measured by DLS (Contin analysis) (See FIG. 4).

The surfactant was dissolved in water (2mL). In a 50 ml centrifuge tube, 20 µl of PTX- or DTX-loaded oil were added, followed by 380 µl of PFOB. The surfactant aqueous solution (2 mL) was added and the mixture was cooled to 0 °C with an ice bath. The mixture was insonated for 2 min in pulse mode (Duty Cycle 11.97%, pulse width 8 s) with a sonicator (VibraCell. TM. 75043, 750 W, Bioblock Scientific, USA), using a 13 mm sonotrode, placed at the bottom of the centrifuge tube, at an amplitude of 60%. The resulting emulsion was centrifuged for 5 min at 5580 g. The supernatant was collected and filtered over a 0.22 µm CA filter, to provide the ND2-PTX or ND2-DTX emulsion. The diameter D2 was assessed by DLS intensity-weighted size distribution (Contin analysis), using a Zetasizer from Malvern Panalytical ^{®} after diluting ten times the emulsion.

The encapsulation efficiency (EE) of drugs was determined by estimating the amount of free drug (fdrug) in the supernatant relative to the total amount of drug (tdrug) in the whole emulsion and was calculated as follows. $% EE = \left[\left(tdrug-fdrug\right)/tdrug\right] * 100$

The emulsion was centrifuged at 25830 g for 45 min, then the fdrug along with the tdrug were titrated by RP-HPLC.

The same freeze-drying conditions were used as for ND2-DiD *(vide supra),* except that 90 µl of trehalose were added (instead of 45 µL used for the freeze-drying of ND2-DiD emulsion). As an example, after freeze-drying a ND2-DTX emulsion (comprising ATBC), a (tdrug) concentration in DTX of 0.98 ± 0.09 mg/ml of emulsion was measured by RP-HLPC, which is a relevant concentration for in vivo testing in mouse animal model.

### c. Preparation of an example of emulsion according to one or more embodiments of the present disclosure "ND1+ND2". Stability in human plasma of ND1, ND2, and "ND1+ND2".

Dry formulations (white powders) of ND1, ND1-FITC, ND2-DiD were resuspended in ionized water (1 mL for ND1 and 300 µL for ND2) to afford the corresponding emulsions. The reconstituted emulsions of ND2-DiD or ND1-FITC (10 µL) were mixed with human plasma (140 µL). The resulting mixture solution was introduced into a 96 well black plate (Corning Life Science, BV). The fluorescence intensity of each mixture (DiD: λEx 648 nm, λEm 670 nm; FITC: λEx 490 nm, λEm 517 nm; human plasma on its own was used as reference) was measured immediately, 5, 10 and 30 min after mixing, using a microplate fluorescence reader (Spectra Mas Gemini Molecular Devices, Sunnyvale, CA, USA).

The fluorescence intensity of each emulsion of fluorescent nanodroplets (ND2-DiD or ND1-FITC) in human plasma was stable over time, suggesting that each individual ND is stable in the presence of human plasma.

10 µL of each ND batch (dry formulation resuspended in water), ND1-FITC and ND2-DiD, were mixed to afford 20 µL of emulsion "ND1 + ND2" (ND1-FITC + ND2-DiD). The emulsion was then diluted in human plasma (130 µL). The fluorescence intensity of ND1-FITC did not change over time in human plasma, indicating that the co-incubation of ND1-FITC with ND2-DiD did not influence the stability of ND1-FITC. Similarly, 10 µL of ND2-DiD were mixed with 10 µL of non-fluorescent ND1. The resulting emulsion "ND1 + ND2-DiD" was then diluted in human plasma (130 µL). As previously observed, the fluorescence intensity of ND2-DiD did not change over time in human plasma, suggesting that the co-mixing of ND2-DiD with non-fluorescent ND1 did not influence the stability of ND2-DiD.

### III. Illustration of the vaporization of the first droplets

The first droplets, *i.e.,* the "large" or "opener" droplets and the second droplets, *i.e.,* the "small" or "drug-loaded" or "carrier" droplets act in synergy in the emulsion or dry formulations of the present disclosure. The acoustic pressure thresholds for achieving the three steps mentioned *vide supra, i.e.,* BBB transient opening, drug vectorization and subsequent controlled drug delivery/release into the brain, may all remain lower than 2 MPa in one or more embodiments of the present disclosure, thereby avoiding or mitigating biological effects that may be induced by US, such as for example neuroinflammation or other damages to the brain parenchyma.

For example, the first US sequence mentioned in part I and illustrated by reference 7, in FIG. 1, left, induces the vaporization of the first droplets 1 into bubbles 1'.

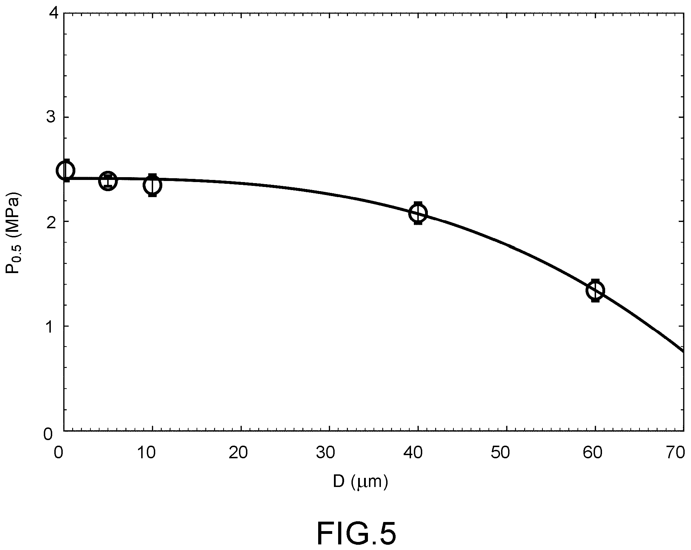

FIG. 5 is a graphical representation of the variation of the peak-to-peak pressure at which the ADV probability is 0.5, with the D1 diameter of first droplets comprising PFH. As shown in FIG. 5, the acoustic pressure of the first focused US 7 beam may then be of no more than 2 MPa while allowing sufficient vaporization of the first droplets 1 into bubbles 1'.

The graph in FIG. 5 was determined in vitro for large droplets of PFC, perfluorohexane (PFH) in this example. The acoustic pressure P_{0.5} at which acoustic droplet vaporization probability Π = 0.5 was determined for different diameters D1 of PFH droplets (0.1 µm; 2.5 µm; 5 µm; 20 µm; 30 µm). For this purpose, 200 µL of each droplet suspension (with its respective diameter D1) were added to a tube placed at the focus of a focused transducer.

The vaporization was then induced by very short insonation, made of 5 cycles sine pulse repeated at a pulse repetition frequency of 11 kHz. The pulse frequency was varied from 0.7 to 3.5 MHz while the pressure amplitude was varied from 0 up to 6 MPa. The focused hydrophone, whose focus overlaps the transducer focus inside the tube, continuously monitored the signal scattered by the sample to detect acoustical droplet vaporization (ADV). The scattered signal was analyzed using a MATLAB code. At each pressure, the probability, Π, of ADV was estimated by counting the number of pulses with subharmonics occurrence over 100 pulses. The ADV threshold probability, Π was fitted to the following formula (1): $Π = 1 - exp \left(-\left(ln 2\right){e}^{α \left(\frac{P}{P 0.5}-1\right)}\right)$ where P is the pressure amplitude, P_{0.5} is the pressure at which the ADV probability is 0.5 and α is a variable.

### IV. Details regarding the measurement of the diameter D and polydispersity index PDI of emulsions by dynamic light scattering (DLS)

The mean radius R (hence, the mean diameter D divided by two) of the spherical droplets forming the emulsion was measured by DLS on diluted solution to avoid interaction between droplets. The DLS device measures the autocorrelation function of the intensity of the laser scattered light. This autocorrelation function is made of a sum of exponentials associated with a decay rate Γ*_{θ}*(*R*), that depends on the droplet radius R and on the angle *θ* at which the measurement is performed, weighted by probability factors. The cumulant method, defined in the International Standard on Dynamic Light Scattering ISO13321 (1996) and ISO22412 (2008), consists in considering a mean decay <Γ*_{θ}*> associated with a mean radius <R> to fit the autocorrelation function. The method also provides the polydispersity index PDI.

These experiments can be performed on multi-angle instruments such as an ALV/CGS-3 platform-based goniometer system (from ALV GmbH) or on a Zeta Sizer Nano-S system (from Malvern instrument). The devices of the first type are capable of performing measurement at several angles (between 12° and 152° for ALV), while the second type of device can only acquire the value <Γ*_{θ}*> at a single angle of (173°). It is better to perform experiment with multi-angle instruments such as ALV goniometer when no information is available on the droplet size distribution. Indeed, this system provides values of <Γ*_{θ}*> for several scattering angles (for instance from 50° to 130°, with a step of 20°). These values can be plotted as a function of the scattering vector amplitude *q*(*θ*) *=* 4 π *n* sin(*θ*/2)/*λ*, where *n* is the refractive index of the solution and *λ* = 633 nm is the laser wavelength of the ALV system. At each angle, we have: $\left〈{Γ}_{θ}\right〉 = \left({k}_{B}T\right) / \left(6πη\left〈R\right〉\right) \times q \left(θ\right)$ where *η* is the viscosity of the solvent.

If there is only one droplet population with a mean radius <R>, then the above linear equation fits the behavior of <Γ*_{θ}*> versus *q*(*θ*), as <R> does not change with the angle. Otherwise, the value of <R> will be dependent on *θ* and a linear fit is not possible.

For solutions produced in a reproducible way, where it has been previously verified that the process for producing the emulsion leads to only one droplet population of mean radius <R>, the Zeta Sizer Nano-S system may be used for practicability.

The examples or embodiments described in this disclosure are illustrative and non-limiting, and a person skilled in the art may easily, in view of this disclosure, modify these examples or embodiments, or consider others, while remaining within the scope of the invention.

In particular, a person skilled in the art will be able to devise variations comprising only some of the features of the previously described examples or embodiments, if these features alone are sufficient to provide one of the advantages of the invention. Furthermore, the various features of these examples or embodiments may be used alone or combined with each other. When combined, these features may be combined as described above or differently, as the invention is not limited to the specific combinations described herein. In particular, unless otherwise specified, a feature described in connection with one example or embodiment may be similarly applied to another example or embodiment.

## Claims

1. An emulsion comprising:
a first discontinuous phase comprising first droplets (1) including at least one first surfactant, and at least one first fluorocarbon, the first droplets having a first mean diameter of more than 100 nm and being substantially or completely free of any drug;
a second discontinuous phase comprising second droplets (2) including at least one second surfactant, at least one drug, and at least one solvent, the second droplets having a second mean diameter of no more than 100 nm; and
a continuous aqueous phase;
wherein the first mean diameter and the second mean diameter are measured by dynamic light scattering (DLS), according to the method described in the present description.

2. The emulsion of claim 1, wherein:
the at least one first fluorocarbon has a boiling point of no more than 100°C; and/or
the first droplets further include a second fluorocarbon having a boiling point of no more than 0°C.

3. The emulsion of claim 1 or claim 2, wherein the at least one first surfactant and/or the at least one second surfactant is/are selected from the group comprising a dendrimer of Dendri-TAC type, an oligomer of F*ᵢ*TAC*ₙ* or H*ᵢ*TAC*ₙ* type, or a mixture thereof.

4. The emulsion of any one of the preceding claims, wherein the at least one first fluorocarbon is a perfluorocarbon, PFC.

5. The emulsion of claim 4, wherein the PFC is selected from the group comprising perfluoropentane (PFP), perfluorohexane (PFH), or a mixture thereof.

6. The emulsion of any one of the preceding claims, wherein:
the first mean diameter is comprised between more 200 nm and 600 nm; and/or
the second mean diameter is comprised between 20 nm and 95 nm.

7. The emulsion of any one of the preceding claims, wherein:
the first droplets (1) have a PDI of no more than 0.5; and/or
the second droplets (2) have a PDI of no more than 0.5;
wherein the PDI of the first droplets (1) and the PDI of the second droplets (2) are measured by dynamic light scattering (DLS), according to the method described in the present description.

8. The emulsion of any one of the preceding claims, wherein the at least one solvent is selected from the group comprising lipophilic compounds, fluorocarbons, or mixtures thereof.

9. The emulsion of any one of the preceding claims, wherein the at least one solvent comprises a PFC selected from the group comprising perfluorooctane, perfluorononane, perfluorodecalin, perfluorooctyl bromide (PFOB), perfluoro-15-crown-5-ether (PFCE), 1,1,1-tris(perfluorotert-butoxymethyl)ethane (TPFBME), or mixtures thereof.

10. The emulsion of claim 8 or claim 9, wherein the at least one solvent comprises a lipophilic compound selected from the group comprising mono-, di-, or tri-esters of glycerol or derivatives thereof, mono-, di-, tri- or tetra-esters of citric acid or derivatives thereof, fatty acids, monoesters of fatty acids, sterol esters, sphingolipids, glycerophospholipids, polyketides, saccharolipids, terpenes, lipid derivatives of prenol, or mixtures thereof.

11. The emulsion of any one of the preceding claims, for use in brain therapy.

12. The emulsion for use according to claim 11, for the treatment of a disease affecting the brain comprising the controlled release/delivery of the at least one drug contained in the second droplets (2) into a brain tissue, wherein the controlled release/delivery is triggered by an external stimulus comprising ultrasounds.

13. The emulsion for use in brain therapy of claim 11 or claim 12, for the treatment of a disease affecting the brain, wherein:
the disease is selected from the group comprising neurodegenerative disorders and brain tumors; and/or
the at least one drug is selected from the group comprising riluzole, anacardic acid, docetaxel (DTX), paclitaxel (PTX), temozolomide, fluorouracil (5-FU), Protoporphyrin IX (PP9).

14. A dry formulation obtained by drying the emulsion of any one of claims 1-10.

## Patentansprüche

1. Emulsion, umfassend:
eine erste diskontinuierliche Phase, umfassend erste Tropfen (1), die mindestens ein erstes Tensid und mindestens ein erstes Fluorkohlenstoff umfassen, wobei die ersten Tropfen einen ersten mittleren Durchmesser von mehr als 100 nm aufweisen und im Wesentlichen oder vollständig frei von einem Arzneimittel sind;
eine zweite diskontinuierliche Phase, umfassend zweite Tropfen (2), die mindestens ein zweites Tensid, mindestens ein Arzneimittel und mindestens ein Lösungsmittel umfassen, wobei die zweiten Tropfen einen zweiten mittleren Durchmesser von nicht mehr als 100 nm aufweisen; und
eine kontinuierliche wässrige Phase;
wobei der erste mittlere Durchmesser und der zweite mittlere Durchmesser durch dynamische Lichtstreuung (DLS) gemäß dem in der vorliegenden Beschreibung beschriebenen Verfahren gemessen werden.

2. Emulsion nach Anspruch 1, wobei:
der mindestens eine erste Fluorkohlenstoff einen Siedepunkt von nicht mehr als 100 °C aufweist; und/oder
die ersten Tropfen ferner einen zweiten Fluorkohlenstoff mit einem Siedepunkt von nicht mehr als 0 °C einschließen.

3. Emulsion nach Anspruch 1 oder Anspruch 2, wobei das mindestens eine erste Tensid und/oder das mindestens eine zweite Tensid aus der Gruppe ausgewählt ist/sind, die ein Dendrimer vom Typ Dendri-TAC, ein Oligomer vom Typ F*ᵢ* TAC*ₙ* oder H*ᵢ* TAC*ₙ* oder eine Mischung davon umfasst.

4. Emulsion nach einem der vorhergehenden Ansprüche, wobei der mindestens eine erste Fluorkohlenstoff ein Perfluorkohlenstoff, PFC, ist.

5. Emulsion nach Anspruch 4, wobei das PFC aus der Gruppe ausgewählt ist, die Perfluorpentan (PFP), Perfluorhexan (PFH) oder eine Mischung davon umfasst.

6. Emulsion nach einem der vorhergehenden Ansprüche, wobei:
der erste mittlere Durchmesser zwischen mehr als 200 nm und 600 nm beträgt; und/oder
der zweite mittlere Durchmesser zwischen 20 nm und 95 nm beträgt.

7. Emulsion nach einem der vorhergehenden Ansprüche, wobei:
die ersten Tropfen (1) einen PDI von nicht mehr als 0,5 aufweisen; und/oder
die zweiten Tropfen (2) einen PDI von nicht mehr als 0,5 aufweisen;
wobei der PDI der ersten Tropfen (1) und der PDI der zweiten Tropfen (2) durch dynamische Lichtstreuung (DLS) gemäß dem in der vorliegenden Beschreibung beschriebenen Verfahren gemessen werden.

8. Emulsion nach einem der vorhergehenden Ansprüche, wobei das mindestens eine Lösungsmittel aus der Gruppe ausgewählt ist, die lipophile Verbindungen, Fluorkohlenstoffe, oder Mischungen davon, umfasst.

9. Emulsion nach einem der vorhergehenden Ansprüche, wobei das mindestens eine Lösungsmittel ein PFC umfasst, das aus der Gruppe ausgewählt ist, die Perfluoroctan, Perfluorononan, Perfluorodecalin, Perfluoroctylbromid (PFOB), Perfluoro-15-Kronen-5-Ether (PFCE), 1,1,1-Tris(perfluorotert-butoxymethyl)ethan (TPFBME), oder Mischungen davon, umfasst.

10. Emulsion nach Anspruch 8 oder Anspruch 9, wobei das mindestens eine Lösungsmittel eine lipophile Verbindung umfasst, die aus der Gruppe ausgewählt ist, die Mono-, Di- oder Triester von Glycerin oder Derivaten davon, Mono-, Di-, Tri- oder Tetraester von Zitronensäure oder deren Derivaten, Fettsäuren, Monoester von Fettsäuren, Sterolester, Sphingolipide, Glyzerophospholipide, Polyketide, Saccharolipide, Terpene, Lipidderivate von Prenol, oder Mischungen davon, umfasst.

11. Emulsion nach einem der vorhergehenden Ansprüche zur Verwendung in der Hirntherapie.

12. Emulsion zur Verwendung nach Anspruch 11, zur Behandlung einer Erkrankung, die das Gehirn betrifft, umfassend die kontrollierte Freisetzung/Abgabe des mindestens einen in den zweiten Tröpfchen (2) enthaltenen Arzneimittels in ein Gehirngewebe, wobei die kontrollierte Freisetzung/Abgabe durch einen externen Stimulus, der Ultraschall umfasst, ausgelöst wird.

13. Emulsion zur Verwendung in der Gehirntherapie nach Anspruch 11 oder Anspruch 12 zur Behandlung einer das Gehirn betreffenden Erkrankung, wobei:
die Krankheit aus der Gruppe ausgewählt wird, die neurodegenerative Erkrankungen und Hirntumore umfasst; und/oder
das mindestens eine Arzneimittel aus der Gruppe ausgewählt ist, die Riluzol, Anakardsäure, Docetaxel (DTX), Paclitaxel (PTX), Temozolomid, Fluorouracil (5-FU), Protoporphyrin IX (PP9) umfasst.

14. Trockenformulierung, die durch Trocknen der Emulsion nach einem der Ansprüche 1 bis 10 erhalten wird.

## Revendications

1. Émulsion comprenant :
une première phase discontinue comprenant des premières gouttelettes (1) comprenant au moins un premier tensioactif et au moins un premier fluorocarbure, les premières gouttelettes ayant un premier diamètre moyen supérieur à 100 nm et étant sensiblement ou totalement exemptes de tout médicament ;
une deuxième phase discontinue comprenant des deuxièmes gouttelettes (2) comprenant au moins un deuxième tensioactif, au moins un médicament et au moins un solvant, les deuxièmes gouttelettes ayant un second diamètre moyen ne dépassant pas 100 nm ; et
une phase aqueuse continue ;
dans laquelle le premier diamètre moyen et le second diamètre moyen sont mesurés par diffusion dynamique de la lumière (DLS), selon le procédé décrit dans la présente description.

2. Émulsion selon la revendication 1, dans laquelle :
l'au moins un premier fluorocarbure a un point d'ébullition ne dépassant pas 100°C ; et/ou
les premières gouttelettes comprennent en outre un second fluorocarbure ayant un point d'ébullition ne dépassant pas 0°C.

3. Émulsion selon la revendication 1 ou 2, dans laquelle l'au moins un premier tensioactif et/ou l'au moins un deuxième tensioactif est/sont choisi(s) dans le groupe comprenant un dendrimère de type Dendri-TAC, un oligomère de type F*ᵢ*TAC*ₙ* ou H*ᵢ*TAC*ₙ* ou un mélange de ceux-ci.

4. Émulsion selon l'une quelconque des revendications précédentes, dans laquelle l'au moins un premier fluorocarbure est un perfluorocarbure, PFC.

5. Émulsion selon la revendication 4, dans laquelle le PFC est choisi dans le groupe comprenant le perfluoropentane (PFP), le perfluorohexane (PFH) ou un mélange de ceux-ci.

6. Émulsion selon l'une quelconque des revendications précédentes, dans laquelle :
le premier diamètre moyen est compris entre plus de 200 nm et 600 nm ; et/ou
le second diamètre moyen est compris entre 20 nm et 95 nm.

7. Émulsion selon l'une quelconque des revendications précédentes, dans laquelle :
les premières gouttelettes (1) ont un PDI ne dépassant pas 0,5 ; et/ou
les deuxièmes gouttelettes (2) ont un PDI ne dépassant pas 0,5 ;
dans laquelle le PDI des premières gouttelettes (1) et le PDI des deuxièmes gouttelettes (2) sont mesurés par diffusion dynamique de la lumière (DLS), selon le procédé décrit dans la présente description.

8. Émulsion selon l'une quelconque des revendications précédentes, dans laquelle l'au moins un solvant est choisi dans le groupe comprenant les composés lipophiles, les fluorocarbures ou des mélanges de ceux-ci.

9. Émulsion selon l'une quelconque des revendications précédentes, dans laquelle l'au moins un solvant comprend un PFC choisi dans le groupe comprenant le perfluorooctane, le perfluorononane, la perfluorodécaline, le bromure de perfluorooctyle (PFOB), le perfluoro-15-couronne-5-éther (PFCE), le 1,1,1-tris(perfluorotert-butoxyméthyl)éthane (TPFBME), ou des mélanges de ceux-ci.

10. Émulsion selon la revendication 8 ou 9, dans laquelle l'au moins un solvant comprend un composé lipophile choisi dans le groupe comprenant les mono-, di- ou tri-esters du glycérol ou leurs dérivés, les mono-, di-, tri- ou tétra-esters de l'acide citrique ou leurs dérivés, les acides gras, les monoesters d'acides gras, les esters de stérols, les sphingolipides, les glycérophospholipides, les polycétides, les saccharolipides, les terpènes, les dérivés lipidiques du prénol, ou des mélanges de ceux-ci.

11. Émulsion selon l'une quelconque des revendications précédentes, pour une utilisation en thérapie cérébrale.

12. Émulsion pour une utilisation selon la revendication 11, pour le traitement d'une maladie affectant le cerveau, comprenant la libération/administration contrôlée de l'au moins un médicament contenu dans les deuxièmes gouttelettes (2) dans un tissu cérébral, où la libération/administration contrôlée est déclenchée par un stimulus externe comprenant des ultrasons.

13. Émulsion pour une utilisation en thérapie cérébrale selon la revendication 11 ou 12, pour le traitement d'une maladie affectant le cerveau, dans laquelle :
la maladie est choisie dans le groupe comprenant les troubles neurodégénératifs et les tumeurs cérébrales ; et/ou
l'au moins un médicament est choisi dans le groupe comprenant le riluzole, l'acide anacardique, le docétaxel (DTX), le paclitaxel (PTX), le témozolomide, le fluorouracile (5-FU), la protoporphyrine IX (PP9).

14. Formulation sèche obtenue par séchage de l'émulsion selon l'une quelconque des revendications 1 à 10.
